# EUROPÄISCHE PATENTANMELDUNG

(11) **EP 4 635 396 A1**
(43) Veröffentlichungstag der Anmeldung: **22.10.2025**
(21) Anmeldenummer: 25170831.9
(22) Anmeldetag: 15.04.2025
(51) Int. Cl.: A61B 1/00, A61B 1/04, G02B 7/00, G02B 26/00

(54) **FILTERWECHSELVORRICHTUNG FÜR EINE ENDOSKOPISCHE KAMERA, KAMERAKOPF FÜR EIN ENDOSKOP UND NACHRÜSTSATZ ZUM NACHRÜSTEN EINES KAMERAKOPFES UND/ODER EINES ENDOSKOPS**

(30) Priorität: 18.04.2024 DE 102024110879
(71) Anmelder: Karl Storz SE & Co. KG, 78532 Tuttlingen (DE)
(72) Erfinder: KIMMI, Maximilian, 78532 Tuttlingen (DE); KROLL, Kai, 78532 Tuttlingen (DE); HUBER, Florian, 78532 Tuttlingen (DE)

(57) **Zusammenfassung**

Die Erfindung betrifft eine Filterwechselvorrichtung für eine endoskopische Kamera, wobei die Filterwechselvorrichtung mindestens ein erstes drehbares Filterrad, ein zweites drehbares Filterrad und ein drehbares erstes Verbindungsrad aufweist, wobei das erste Filterrad und das zweite Filterrad jeweils mindestens zwei Aufnahmen für jeweils einen optischen Filter aufweisen, die Filterräder eine gemeinsame erste Rotationsachse aufweisen, das zweite Filterrad eine erste Eingriffsebene mit vollständig über seinem Außenumfang verteilten Zähnen aufweist und das erste Verbindungsrad als Zahnrad mit vollständig über seinem Außenumfang verteilten Zähnen ausgebildet ist, wobei das erste Filterrad einen Teilabschnitt mit verteilten Zähnen entlang seines Außenumfanges aufweist und das erste Verbindungsrad an dem Außenumfang des ersten Filterrades und an dem Außenumfang des zweiten Filterrades derart angeordnet ist, dass die Zähne des Teilabschnittes des ersten Filterrades in die Zähne der ersten Verbindungsrades zum Drehen des ersten Verbindungsrades und die Zähne des ersten Verbindungsrades in die Zähne der ersten Eingriffsebene des zweiten Filterrades eingreifbar sind, sodass bei dem Antreiben des ersten Filterrades mittels der Antriebseinheit mindestens jeweils eine Aufnahme des ersten Filterrades und des zweiten Filterrades parallel in den optischen Durchgang positionierbar sind. Des Weiteren betrifft die Erfindung einen Kamerakopf für ein Endoskop und einen Nachrüstsatz zum Nachrüsten eines Kamerakopfes und/oder eines Endoskops.

## Beschreibung

Die Erfindung betrifft eine Filterwechselvorrichtung für eine endoskopische Kamera, wobei die Filterwechselvorrichtung eine Grundplatte, einen optischen Durchgang mit einer optischen Achse, mindestens ein erstes drehbares Filterrad, ein zweites drehbares Filterrad und ein drehbares erstes Verbindungsrad sowie eine Antriebseinheit zum Antreiben eines der Filterräder aufweist, wobei das erste Filterrad und das zweite Filterrad jeweils mindestens zwei Aufnahmen für jeweils einen optischen Filter aufweisen, die Filterräder eine gemeinsame erste Rotationsachse aufweisen, das zweite Filterrad eine erste Eingriffsebene mit vollständig über seinen Außenumfang verteilten Zähnen aufweist und das erste Verbindungsrad als Zahnrad mit vollständig über seinen Außenumfang verteilten Zähnen ausgebildet ist. Des Weiteren betrifft die Erfindung einen Kamerakopf für ein Endoskop und einen Nachrüstsatz zum Nachrüsten eines Kamerakopfes und/oder eines Endoskops.

In medizinischen und nicht-medizinischen Anwendungen werden Beobachtungsinstrumente wie Endoskop eingesetzt, um innere Hohlräume eines menschlichen oder tierischen Körpers oder eines industriellen, technischen Objektes, wie eine Rohrleitung, zu untersuchen. Für die Bildgebung kann ein Kamerakopf aufweisend einen Bildsensor zusammen mit dem Endoskop eingesetzt werden. Um die Bildqualität zu verbessern und/oder verschiedene Beobachtungsmodi zu ermöglichen, ist es bekannt, verschiedene Filter in den Strahlengang des Beobachtungsinstrumentes einzubringen.

Zum Beispiel wird in der Fluoreszenz-Bildgebung das zu untersuchende Objekt einem Licht mit einer Anregungsstrahlung ausgesetzt, welche ein vorher auf das Objekt aufgebrachtes oder bereits vorhandenes Fluorophor anregt, Licht einer bestimmten Emissionswellenlänge auszusenden, wobei die Anregungswellenlänge und die Emissionswellenlänge sich üblicherweise unterscheiden. Normalerweise ist die Emissionswellenlänge länger als die Anregungswellenlänge. Das abgegebene Emissionslicht ist üblicherweise deutlich schwächer als andere Lichtquellen, wie das Anregungsfluoreszenzlicht oder das bildgebende Weißlicht. Aus diesen Gründen ist es notwendig, unerwünschte Wellenlängenbänder mittels eines Filters herauszufiltern, sodass möglichst nur das gewünschte Spektrum und/oder die Emissionswellenlänge des Fluorophors den Kamerakopf im Fluoreszenzmodus erreicht. Neben einem Wechsel zwischen verschiedenen Beobachtungsmodi, bei dem üblicherweise zwei oder mehrere Filter nacheinander in den Strahlengang der Beobachtungsoptik eingebracht werden, kann es auch vorteilhaft sein, zwei verschiedene Filter gleichzeitig in den Strahlengang einzubringen. Hierzu sind prinzipiell verschiedene Filterwechsler bekannt.

DE 10 2020 100 676 B3 offenbart eine Filterwechselvorrichtung für ein optisches Beobachtungsinstrument mit zwei Strahlengängen, welche drei Filterrädern aufweist, die entlang einer gemeinsamen Achse hintereinander angeordnet und um diese gemeinsame Achse und zueinander rotierbar sind, wobei jedes Filterrad mindestens einen Filter und mindestens einen freien optischen Durchgang aufweist, sodass in jedem der beiden Strahlengänge ein Filter oder ein freier optischer Durchgang eines jeden Filterrades einbringbar ist. Das zweite Filterrad ist antreibbar und das erste Filterrad ist über einen ersten Mitnehmer und das dritte Filterrad über einen zweiten Mitnehmer mit dem zweiten Filterrad gekoppelt. Nachteilig hierbei ist, dass aufgrund des mittleren zweiten angetriebenen Filterrades und der Verbindung der beidseitig angeordneten ersten und dritten Filterräder jeweils über einen Mitnehmer die Anzahl der hintereinander in Reihe anordenbaren Filterräder auf drei beschränkt ist.

Des Weiteren ist als Kunstobjekt ein mechanischer Binärzähler von der "Anstalt für freie Kunstmaschinen/Felix Scharstein" (www.scharstein.de/#binaerzaehler) bekannt. Dieser Binärzähler beruht auf einem Mechanismus rein binärer Art, bei dem für jede Umdrehung eines mittels einer Welle angetriebenen Zahnrades genau zwei Positionen vorgesehen sind. Die Zahlentafeln des mechanischen Binärzählers sind entlang einer Rotationsachse mit dazwischen angeordneten Zahnrädern angeordnet, wobei diese Zahnräder in eine zweite Reihe von Zahnrädern teilübertragend eingreifen, welche auf einer zweiten Rotationsachse dahinter angeordnet sind. Die sich jeweils gegenüberliegenden Tafelflächen sind entlang der Rotationsachse angeordnet und nehmen somit in Längsrichtung der Rotationsachse einen hohen Raumbedarf ein. Zudem benötigt dieser mechanische Binärzähler eine hohe Anzahl an Einzelteilen, wodurch der Mechanismus störanfällig wird.

Aufgabe der Erfindung ist es, den Stand der Technik zu verbessern.

Gelöst wird die Aufgabe durch eine Filterwechselvorrichtung für eine endoskopische Kamera, wobei die Filterwechselvorrichtung eine Grundplatte, einen optischen Durchgang mit einer optischen Achse, mindestens ein erstes drehbares Filterrad, ein zweites drehbares Filterrad und ein drehbares erstes Verbindungsrad sowie eine Antriebseinheit zum Antreiben eines der Filterräder aufweist, wobei das erste Filterrad und das zweite Filterrad jeweils mindestens zwei Aufnahmen für jeweils einen optischen Filter aufweisen, die Filterräder eine gemeinsame erste Rotationsachse aufweisen, das zweite Filterrad eine erste Eingriffsebene mit vollständig über seinen Außenumfang verteilten Zähnen aufweist und das erste Verbindungsrad als Zahnrad mit vollständig über seinen Außenumfang verteilten Zähnen ausgebildet ist, wobei das erste Filterrad einen Teilabschnitt mit verteilten Zähnen entlang seinen Außenumfanges aufweist und das erste Verbindungsrad an dem Außenumfang des ersten Filterrades und an dem Außenumfang des zweiten Filterrades derart angeordnet ist, dass die Zähne des Teilabschnittes des ersten Filterrades in die Zähne des ersten Verbindungsrades zum Drehen des ersten Verbindungsrades und die Zähne des ersten Verbindungsrades in die Zähne der ersten Eingriffsebene des zweiten Filterrades eingreifbar sind, sodass bei dem Antreiben des ersten Filterrades mittels der Antriebseinheit mindestens jeweils eine Aufnahme des ersten Filterrades und des zweiten Filterrades parallel in den optischen Durchgang positionierbar sind.

Somit ist eine Filterwechselvorrichtung bereitgestellt, mit welcher zwei oder mehrere Filter parallel und gleichzeitig in den Strahlengang einer endoskopischen Kamera und/oder eines Endoskops präzise und effizient ein- und ausschwenkbar sind. Es ist besonders vorteilhaft, dass zum Antreiben nur eine einzige Antriebseinheit notwendig ist, diese eine Antriebseinheit auch nur auf ein Filterrad einwirkt und dieses Filterrad in Rotation versetzt, wodurch über die Kopplung des angetriebenen Filterrades mit dem benachbarten Filterrad über das Verbindungsrad das benachbarte Filterrad selbst auch in Rotation versetzbar ist, solange die zugehörigen Zähne ineinander eingreifen. Dadurch, dass die mindestens zwei Filterräder in Reihe entlang der gemeinsamen Rotationsachse und somit mit ihren jeweiligen gegenüberliegenden Radflächen quer zur Rotationsachse angeordnet sind, sind die Filterräder beliebig vollübertragend und/oder teilübertragend im Uhrzeigersinn und gegen den Uhrzeigersinn rotierbar, wodurch parallel einzuschwenkende optische Filter in den jeweiligen Aufnahmen beliebig kombinierbar sind. Vor allem sind durch das einfache Rotieren im Uhrzeigersinn und/oder gegen den Uhrzeigersinn eine beliebige Abfolge und ein schneller Wechsel zwischen verschiedenen Filtern und/oder Filterkombinationen und somit schnell nacheinander verschiedene Beobachtungsmodi und Bildgebungen ermöglicht.

Durch die Aneinanderreihung der Filterräder entlang der Rotationsachse, wobei ihre Radflächen quer zur Rotationsachse angeordnet sind, wird eine kompakte Baugröße der Filterwechselvorrichtung bereitgestellt. Durch die kompakte Baugröße ist die Filterwechselvorrichtung einfach in einen Kamerakopf integrierbar oder als kompakter Verbinder zwischen einem Kamerakopf und einem Endoskop nutzbar. Hierbei ist die Abmessung der Filterwechselvorrichtung nicht nur in Längsrichtung durch Aneinanderreihung der Filterräder mit ihrer geringeren Materialstärke entlang der Rotationsachse minimiert, sondern auch in Querrichtung verkleinerbar. Die Filterräder müssen zwar einen ausreichenden Durchmesser für die gewünschte Anzahl an Aufnahmen der optischen Filter aufweisen. Ein jeweiliges Verbindungsrad hat jedoch nur die Funktion, die Rotationsbewegung des vorhergehenden Filterrades auf das nachfolgende Filterrad zu übertragen. Dadurch ist der Durchmesser eines jeweiligen Verbindungsrades deutlich geringer ausbildbar als der Durchmesser der jeweiligen Filterräder, sodass der Gesamtdurchmesser der Filterwechselvorrichtung minimierbar ist.

Dadurch, dass das erste Filterrad und/oder ein endständiges Filterrad einen Teilabschnitt mit verteilten Zähnen entlang seinen Außenumfanges aufweist, wird bei Antreiben dieses Filterrades durch Eingreifen der Zähne dieses ersten Teilabschnittes des ersten Filterrades in die Zähne des ersten Verbindungsrades das Verbindungsrad in Rotation versetzt. Die Zähne des ersten Verbindungsrades greifen dabei wiederum in die Zähne der ersten Eingriffsebene des zweiten Filterrades mit vollständig über seinen Außenumfang verteilten Zähnen. Somit wird bei einer vollen Umdrehung des ersten Filterrades aufgrund des Teilabschnittes mit den Zähnen über das erste Verbindungsrad eine halbe Umdrehung des zweiten Filterrades bewirkt. Durch diesen mechanischen, ursprünglich binären Mechanismus sind verschiedene Kombinationen an optischen Filtern mit nur einer einzigen Antriebseinheit in den Strahlengang gleichzeitig einschwenkbar und anordenbar. Pro Zahnrad ist jeweils ein optischer Filter einschwenkbar und somit sind mindestens zwei optische Filter gleichzeitig und parallel in den Strahlengang einschwenkbar und positionierbar.

Ein wesentlicher Gedanke der Erfindung beruht darauf, dass bei aneinandergereihten Filterrädern alleinig das erste und/oder endständige Filterrad antreibbar oder angetrieben ist und aufgrund eines Teilabschnittes mit Zähnen, welcher nicht den vollständigen Außenumfang des ersten Filterrades einnimmt, nur bei Eingreifen der rundumlaufenden Zähne eines Verbindungsrades in diesen Teilabschnitt über das Verbindungsrad die Drehbewegung auf das nachfolgende zweiter Filterrad übertragen wird und somit das zweite Filterrad sich ebenfalls dreht und eine entsprechende Filteraufnahme des zweiten Filterrades in den Strahlengang parallel einschwenkbar ist. Wenn der Umfangsabschnitt frei von dem ersten Teilabschnitt mit den Zähnen des ersten Filterrades zum ersten Verbindungsrad ausgerichtet ist, findet kein Eingreifen zwischen dem ersten Filterrad und dem ersten Verbindungsrad statt und das erste Verbindungsrad und folglich auch das zweite Filterrad bleiben stehen. Somit ist ein mechanischer binärer Übertragungsmechanismus realisiert, bei dem beim Drehen des ersten und/oder vorherigen Filterrades das zweite und/oder nachfolgende Filterrad entweder stehenbleibt oder selbst gedreht wird. Dadurch ist jeweils eine Aufnahme der mindestens zwei Filterräder in dem Strahlendurchgang angeordnet und somit sind mindestens zwei Filter parallel in den Strahlendurchgang eingeschwenkt. Folglich wird eine Filtervorrichtung bereitgestellt, mit welcher mindestens zwei gleiche und/oder verschiedene Filter, insbesondere Fluoreszenzfilter, in den Strahlengang einer endoskopischen Kamera präzise, schnell, effizient und vor allem gleichzeitig mit einer langen Standzeit aufgrund des rein mechanischen Übertragungsmechanismus ein- und ausschwenkbar sind. Neben dem schnellen Filterwechsel ist durch das Einsetzen von gleichen und/oder verschiedenen Filtern in die Aufnahmen auch die Häufigkeit der Verwendung eines bestimmten Filters vorgebbar. Beispielsweise wird üblicherweise ein Beobachtungsmodus mit normalen Weißlicht häufiger verwendet als Fluoreszenzlicht. Vorteilhaft ist somit ein Weißlichtfilter jeweils vor und/oder nach einem Fluoreszenzfilter in nacheinander folgenden Aufnahmen eines Filterrades oder versetzt zwischen den Aufnahmen von zwei oder mehreren Filterrädern anordenbar, sodass neben einer gewünschten häufigeren Nutzung von Weißlich durch den jeweiligen Wechsel auf ein Weißlichtfilter dem Anwender auch eindeutig optisch der Wechsel zwischen zwei gleichen oder verschiedenen aufeinanderfolgenden anderen Filtern, beispielsweise zwei Fluoreszenzfiltern, anzeigbar ist. Somit wird das Risiko vermindert, dass unbeabsichtigt in einem falschen Beobachtungsmodus gearbeitet wird.

Folgendes Begriffliche sei erläutert:
Eine "Filterwechselvorrichtung" ist insbesondere eine Vorrichtung, mit welcher mindestens zwei Filter parallel und gleichzeitig in oder aus dem optischen Strahlengang bewegbar und/oder im optischen Durchgang anordenbar sind. Die Filterwechselvorrichtung ist insbesondere manuell oder automatisch mittels einer Antriebseinheit aktivierbar zum parallelen Wechseln mindestens zweier Filter durch Drehen des ersten und/oder eines endständigen Filterrades. Somit ist ein Filter oder sind zwei oder mehrere Filter automatisch oder manuell durch Drehen in den optischen Durchgang ein- und ausschwenkbar. Die Filterwechselvorrichtung weist mindestens ein erstes Filterrad und ein zweites Filterrad sowie mindestens ein erstes Verbindungsrad auf, wobei das erste Verbindungsrad mit seinen rundumlaufenden Zähnen sowohl in die Zähne des Teilabschnittes des ersten Filterrades und die Zähne der ersten Eingriffsebene des zweiten Filterrades eingreifbar ist. Dementsprechend ist das Verbindungsrad vorzugsweise an den Außenumfängen des ersten Filterrades und des zweiten Filterrades angeordnet. Die kompakt bauende Filterwechselvorrichtung kann insbesondere in einer Kamera integriert sein oder als separate Vorrichtung, beispielsweise ausgebildet als Aufschnappfilter, mit der Kamera und/oder einem Endoskop verbindbar sein. Für einen automatischen Filterwechsel kann die Filterwechselvorrichtung ein Bedienelement, beispielsweise einen Schalter an ihrer Außenoberfläche, aufweisen. Alternativ oder ergänzend zu einer optischen Erkennung des Filterwechsels durch den Anwender kann die Filterwechselvorrichtung auch ein Anzeigeelement und/oder einen Sensor, beispielsweise einen Hall-Sensor, aufweisen.

Eine "Grundplatte" ist insbesondere ein Bestandteil der Filterwechselvorrichtung, auf und/oder an welchem die mindestens eine Rotationsachse innenliegend angeordnet ist. Bei einer Grundplatte kann es sich auch um ein Gehäuseteil und/oder einen Gehäusedeckel der Filterwechselvorrichtung handeln.

Ein "Filterrad" (auch "Wechselscheibe" genannt) ist insbesondere eine Scheibe, welche mindestens zwei Aufnahmen für jeweils einen optischen Filter aufweist. Das Filterrad kann insbesondere als Zahnrad ausgebildet sein. Die Aufnahmen sind insbesondere gleichmäßig zueinander entlang eines inneren Umfanges des Filterrades und/oder verteilt über die Radfläche des Filterrades angeordnet. Das jeweilige Filterrad ist insbesondere um die Rotationsachse drehbar. Das erste Filterrad und/oder eines der beiden endständigen Filterräder kann insbesondere per Hand und/oder mittels einer Antriebseinheit und/oder einem Motor angetrieben und gedreht werden. Dazu kann beispielsweise die äußere Umfangsfläche als Kontaktfläche angetrieben werden, indem eine Antriebseinheit und/oder ein Getriebe an dieser oder/oder auf diese Kontaktfläche einwirkt. Beispielsweise greift der Antrieb hierbei in eine zusätzliche Ebene des ersten Filterrades und/oder des endständigen Filterrades ein, welche vollständig rundumlaufende Zähne aufweist zusätzlich zu dem ersten Teilabschnitt in einer anderen Ebene. Bevorzugt ist jedoch das erste Filterrad und/oder das endständige Filterrad mit einer Rotationswelle verbunden und wird durch Drehen der Rotationswelle mittels einer Antriebseinheit oder per Hand angetrieben. Das jeweilige Filterrad ist insbesondere ein flaches ebenes Bauteil, wobei dessen gegenüberliegende Radflächen im Wesentlichen senkrecht zur optischen Achse und/oder zur Rotationsachse ausgerichtet sind. Somit sind mindestens zwei Filterräder oder mehrere Filterräder mit ihren gegenüberliegenden Radflächen jeweils zueinander und insbesondere quer zur Rotationsachse ausgerichtet. Dadurch sind die Filterräder mit ihren jeweiligen Materialdicken aneinandergereiht entlang der Rotationsachse angeordnet. Das jeweilige Filterrad ist insbesondere als rundes Rad und/oder Scheibe ausgebildet. Bevorzugt ist das jeweilige Filterrad in seinem Querschnitt kreisrund ausgebildet. Das Filterrad kann jedoch auch eine elliptische Querschnittsform aufweisen und somit als Ellipsenrad ausgebildet sein.

Das jeweilige Filterrad kann an seinem Außenumfang vollständig umlaufend verteilte Zähne und/oder einen Zahnradkranz aufweisen oder weist lediglich einen Teilabschnitt mit verteilten Zähnen entlang seines Außenumfangs auf. Die gleichmäßig verteilten Zähne eines Filterrades können entlang der Materialdicke des Filterrades und somit entlang der optischen Achse und/oder Rotationsachse homogen, durchgehend ausgebildet sein oder die Zähne sind in einer Ebene angeordnet und somit sind die Zähne nicht vollständig über die Materialdicke des Zahnrades ausgebildet. Somit kann ein jeweiliges Filterrad über seinen gesamten Umfang und seine gesamte Materialdicke gleichmäßig verteilte Zähne aufweisen. Ebenso kann ein Filterrad über einen Teilabschnitt seines Außendurchmessers und vollständig über seine Materialdicke gleichmäßig verteilte Zähne aufweisen. Auch kann ein jeweiliges Filterrad in einer ersten Eingriffsebene vollständig entlang seines Außendurchmessers gleichmäßig verteilte Zähne aufweisen und in einer entlang der Rotationsachse und/oder optischen Achse nachfolgenden zweiten Eingriffsebene an seiner Stirn- und/oder Außenfläche einen Teilabschnitt mit den gleichmäßig verteilten Zähnen aufweisen, wobei die Zähne der ersten Eingriffsebene und der zweiten Eingriffsebene mit dem Teilabschnitt insbesondere gleichartig und/oder durchgehend entlang der Materialdicke ausgebildet sind. Der Abschnitt der Außenfläche des jeweiligen Zahnrades und/oder die jeweilige Eingriffsebene, welche frei von Zähnen ist, weist in diesem Bereich frei von Zähnen insbesondere einen geringeren Durchmesser als ein Teilabschnitt und/oder eine Eingriffsebene mit verteilten Zähnen auf. Somit kann der Abschnitt entlang des Außenumfanges frei von Zähnen als Aussparung und/oder Austragung ausgebildet sein. Somit kann ein jeweiliges Filterrad mit einer ersten Eingriffsebene ausgebildet als komplettes Zahnrad und einer zweiten Eingriffsebene ausgebildet als Teilzahnrad sein.

Die Filterräder weisen insbesondere jeweils eine Bohrung teilweise oder vollständig durch ihre Materialdicke zum Ein- und/oder Durchführen der ersten Rotationsachse auf. Die jeweilige Bohrung ist insbesondere mittig zum Durchmesser des jeweiligen Filterrades angeordnet. Die erste Rotationsachse für die Filterräder ist insbesondere innenliegend im Gehäuse der Filterwechselvorrichtung, an ihrer Grundplatte und/oder einem Gehäusedeckel befestigt. Da bevorzugt der optische Durchgang durch das Gehäuse der Filterwechselvorrichtung mittig zum Außendurchmesser des Gehäuses angeordnet ist, ist die erste Rotationsachse der Filterräder und/oder die zweite Rotationsachse des Verbindungsrades oder der Verbindungsräder entsprechend weiter außen und außermittig angeordnet. Das erste Filterrad und/oder ein endständiges Filterrad kann insbesondere eine Welle zum Antreiben aufweisen, wobei die Welle an der Seite der Radfläche angeordnet ist, welche gegenüberliegend zu der Seite ausgerichtet zum zweiten Filterrad ist.

Durch die Ausbildung des ersten Filterrades mit einem Teilabschnitt mit verteilten Zähnen und Eingreifen dieser Zähne in die Zähne des ersten Verbindungsrades wird mittels des ersten Verbindungsrades bei einer vollständigen Umdrehung des ersten Filterrades entsprechend nur eine Teildrehung auf das mit dem ersten Verbindungsrad ebenfalls im Eingriff stehende zweite Filterrad übertragen. Somit ist das erste Filterrad und/oder ein endständiges Filterrad insbesondere nur teilübertragend auf das erste Verbindungsrad und das gekoppelte zweite Filterrad. Die maximale Rotationsdistanz bei einer ganzen Umdrehung des Filterrades, um eine gewünschte Filterkombination zu erreichen, ist (2ⁿ)/2 mit n = Anzahl der Filterräder.

Bei einem "Verbindungsrad" (auch "Hilfszahnrad") handelt es sich insbesondere um ein Zahnrad. Das Verbindungsrad weist insbesondere über den Umfang und/oder seine Materialdicke gleichmäßig verteilte Zähne auf. Das Verbindungsrad ist insbesondere als Stirnrad und/oder Zylinderrad ausgebildet. Die Rotationsachse des Verbindungsrades ist insbesondere parallel zur Rotationsachse der Filterräder. Das jeweilige Verbindungsrad und die beiden Filterräder, in welchen dieses Verbindungsrad eingreift, bilden insbesondere ein Stirnradgetriebe aus. Ein Verbindungsrad weist insbesondere einen deutlich geringeren Durchmesser als der Durchmesser einer Filteraufnahme auf. Zwei oder mehrere Verbindungsräder können auf einer gemeinsamen Rotationsachse angeordnet sein. Dazu weist das jeweilige Verbindungsrad insbesondere eine mittige durchgehende Bohrung zum Durchführen der Rotationsachse auf. Die Rotationsachse kann innenliegend am Gehäuse der Filterwechselvorrichtung, beispielsweise auf einer Grundplatte oder einem Gehäusedeckel, befestigt sein.

Die "erste Rotationsachse" ist insbesondere als gemeinsame Achse für die rotierbaren Filterräder und die "zweite Rotationsachse" als gemeinsame Achse für die Verbindungsräder ausgebildet. Unter einer "gemeinsamen Rotationsachse" wird insbesondere ein Maschinenelement zur Lagerung der rotierbaren Filterräder oder Verbindungsräder verstanden. Die gemeinsame Rotationsachse rotiert insbesondere nicht selbst und überträgt somit kein Drehmoment. Die feststehende gemeinsame Rotationsachse bildet somit auch eine gemeinsame Drehachse der Filterräder oder der Verbindungsräder ab. Über die gemeinsame Rotationsachse werden insbesondere auch eine aneinanderreihende Anordnung der Filterräder oder der Verbindungsräder entlang der gemeinsamen Rotationsachse sichergestellt. Dazu weist jedes Filterrad bevorzugt eine mittige Bohrung auf, sodass die gemeinsame Rotationsachse durch die mittigen Bohrungen der Filterräder geführt ist und somit die Filterräder "fluchtend" in einer geraden Linie mit ihrem Mittelpunkt liegen.

Prinzipiell ist herauszustellen, dass die Bezeichnungen "erstes" und "zweites" Filterrad oder "erster" und "zweiter" Teilabschnitt, Filter und weitere Begriffe nur der Unterscheidung dienen und nicht zwingend eine Reihenfolge vorgeben.

Eine "Aufnahme" (auch "Filteraufnahme" genannt) ist insbesondere ein Hohlraum und/oder ein in eine Öffnung innerhalb des jeweiligen Filterrades einsetzbarer Hohlkörper. Die Aufnahme kann insbesondere außen eine teilweise oder vollständige Umfassung und/oder Umrandung aufweisen, in welchen ein optisches Filter einsetzbar ist und welche den optischen Filter an seinem Umfang zumindest teilweise umschließt. Eine Aufnahme kann beispielsweise ein kurzer rohrförmiger Körper sein. Die Aufnahme bildet insbesondere eine Schutzhülle für den optischen Filter. Die Aufnahme kann aber auch direkt als durchgehende Öffnung, beispielweise ausgebildet als Bohrung, durch die Materialstärke des jeweiligen Filterrades ausgebildet sein. Optional kann das jeweilige Filterrad auch eine Filteraufnahme aufweisen, welche kein optisches Filter aufweist und somit einen freien Durchlass durch den optischen Strahlengang ermöglicht. Somit kann mittels des jeweiligen Filterrad auch eine leere Filteraufnahme in den optischen Durchgang und in den Strahlengang eingebracht werden. Ebenso kann der freie Durchgang statt Weglassen eines optischen Filters auch durch ein nicht filtrierendes optisches Element, wie eine Glasscheibe, ermöglicht sein. Eine Glasscheibe als Fenster kann auch eine Anti-Reflexions-Beschichtung aufweisen.

Ein "optischer Filter" (vereinfacht auch als "Filter" bezeichnet) ist insbesondere ein optisches Element, welches die einfallende Strahlung und/oder einfallenden Strahlen basierend auf spezifischen Eigenschaften, wie beispielsweise einer Wellenlänge, einem Polarisationszustand, einem Einfallswinkel und/oder einer Einfallsrichtung, selektiert und somit durchlässt oder am Durchlassen hindert. Ebenso kann ein optisches Filter die Eigenschaften des durchtretenden Lichtes verändern, beispielsweise durch Umwandlung von kreisförmig polarisiertem Licht in linear polarisiertem Licht. Insbesondere kann ein optischer Filter ein spezifisches spektrales Wellenlängenband blockieren. Ein optischer Filter kann beispielsweise ein Verlaufsfilter, ein Kantenfilter, ein Polarisationsfilter oder ein Interferenzfilter sein. Ein Interferenzfilter weist insbesondere eine Beschichtung auf, welche ein Blockieren oder Durchlassen von Licht eines bestimmten Spektralbereiches bewirkt. Der optische Filter ist insbesondere als Beobachtungsfilter und/oder Detektionsfilter, Fluoreszenzbeobachtungsfilter oder Anregungsfilter einsetzbar. Der optische Filter weist insbesondere Glas oder ein kristallines Material auf. Der optische Filter kann planar oder als Filterlinse ausgebildet sein. Prinzipiell kann anstelle des optischen Filters auch ein anderes optisches Element, wie beispielsweise eine Linse, eine Blende, ein Polarisator oder ein ähnliches optisches Element, in der Filterwechselvorrichtung und/oder der Filteraufnahme angeordnet sein.

Unter "Weißlichtfilter" wird insbesondere verstanden, dass eine entsprechende Aufnahme und/oder Position frei von einem optischen Element ist oder ein optisches Element in einer entsprechenden Aufnahme und/oder Position frei von einer Filterfunktion ist, sodass das Licht und/oder Weißlicht insbesondere unverändert durchgelassen wird. Weißlicht wird von einem Weißlichtfilter insbesondere ohne Änderung seiner Lichteigenschaften, insbesondere der Wellenlängen, durchgelassen. Bei einem Weißlichtfilter kann es sich auch um ein Filter handeln, welches Nahinfrarotes Licht blockt. Ein "Weißlichtfilter" kann auch ein Filter sein, welcher das Licht filtriert, um eine Qualität des Bildes bei Beleuchtung mit weißem Licht zu verbessern. Hierfür kann z.B. ein BG39 Filter von der Firma Schott verwendet werden. Somit kann mittels eines Weißlichtfilters auch das durch einen Bildsensor und/oder eine Kamera erfasste Licht an eine Empfindlichkeitskurve und/oder eine spezifische Empfindlichkeit des menschlichen Auges angepasst werden.

Ein "Fluoreszenzbeobachtungsfilter" (auch als "Fluoreszenzfilter" bezeichnet) ist insbesondere ein optisches polychroitisches Interferenzfilter zum Trennen des emittierten Fluoreszenzlichtes von dem eingesetzten Anregungslicht. Somit blockiert das Fluoreszenzfilter die spezifische Fluoreszenzanregungsstrahlung und lässt die Fluoreszenzemissionsstrahlung entlang des optischen Strahlengangs durch. Bevorzugt blockiert das Fluoreszenzfilter vollständig das Anregungslicht, während es das Fluoreszenzemissionslicht durchlässt, welches üblicherweise eine längere Wellenlänge als das Anregungslicht aufweist. Somit handelt es sich bei einem Fluoreszenzfilter insbesondere um ein Beobachtungsfilter, welcher das Anregungslicht, welches ein Fluorophor zum Leuchten bringt, herausfiltert. Dies ist vorteilhaft, da das Anregungslicht in der Regel mehrere Größenordnungen heller ist als das resultierende und/oder emittierte Fluoreszenzlicht und dieses sonst überstrahlt. Bei einem Fluoreszenzfilter kann es sich auch um ein "Blaufilter", "Rotfilter", "IR-Filter" oder "NIR-Filter" handeln.

Unter "Blaufilter" wird insbesondere ein Filter verstanden, welcher das blaue Anregungslicht einer Lichtquelle herausfiltert, aber das Fluoreszenzlicht, insbesondere ein von einem Fluorophor emittiertes Fluoreszenzlicht, zumindest überwiegend durchlässt. Beispielsweise erfolgt bei der Verwendung des Fluorophor FITC (Fluorescein isothiocyanat, grünes Derivat von Fluorescein) eine Anregung mittels LED bei einer Wellenlänge von 460 nm, wobei langwelligeres Fluoreszenzlicht mit einem Maximum bei ca. 520 nm im grünen Spektralbereich vom Fluorophor emittiert wird. Um das emittierte Fluoreszenzlicht des FITC in der Bildgebung gut darstellen zu können, wird mittels der Filterwechselvorrichtung und/oder Kamera das blaue Anregungslicht herausgefiltert.

Unter "Rotfilter" wird insbesondere ein Filter verstanden, welcher das rote Anregungslicht einer Lichtquelle herausfiltert, aber das Fluoreszenzlicht, insbesondere ein von einem Fluorophor emittiertes Fluoreszenzlicht, zumindest überwiegend durchlässt.

Unter "IR-Filter" wird insbesondere ein Filter verstanden, welcher infrarotes Anregungslicht einer Lichtquelle herausfiltert, aber das Fluoreszenzlicht, insbesondere ein von einem Fluorophor emittiertes Fluoreszenzlicht, zumindest überwiegend durchlässt.

Unter "NIR-Filter" wird insbesondere ein Filter verstanden, welcher nahinfrarotes Anregungslicht einer Lichtquelle herausfiltert, aber das Fluoreszenzlicht, insbesondere ein von einem Fluorophor emittiertes Fluoreszenzlicht, zumindest überwiegend durchlässt.

Ein "optischer Durchgang" ist insbesondere ein ausgesparter Raum in der Filterwechselvorrichtung, durch welchen Licht durchtreten kann. Ein optischer Durchgang ist insbesondere eine durchgehende Öffnung durch die Filterwechselvorrichtung, deren weiteren Bestandteilen und/oder einem Gehäuses der Filterwechselvorrichtung. Der optische Durchgang ist insbesondere um den Mittelpunkt des Querschnittes der drehbaren Filterräder und/oder um die optische Achse angeordnet. Der optische Durchgang erstreckt sich insbesondere entlang der optischen Achse. In Lichtausbreitungsrichtung vor und/oder in dem optischen Durchgang kann insbesondere eine Filteraufnahme und/oder ein optisches Filter angeordnet sein. Ebenso kann der optische Durchgang bei Lichtdurchtritt frei von einem angeordneten optischen Filter und/oder einer Aufnahme sein. Prinzipiell kann der optische Durchgang sämtliche Querschnittsformen aufweisen, bevorzugt ist der optische Durchgang kreisrund im Querschnitt ausgebildet.

Eine "optische Achse" ist insbesondere eine Linie, entlang der ein Grad an Rotationssymmetrie in einem optischen System existiert. Die optische Achse ist insbesondere eine imaginäre Linie, welche einen Pfad definiert, entlang dem Licht durch die Filterwechselvorrichtung und/oder die Kamera in Richtung eines Bildsensors propagiert. Vorzugsweise läuft die optische Achse durch den Krümmungsmittel des jeweils eingeschwenkten Filters und/oder eines nachgeschalteten Linsensystems und/oder Objektivsystems. Die optische Achse kann jedoch auch durch eine Linse, ein optisches Element und/oder eines der optischen Filter gebogen und/oder gelenkt werden. Der optische Strahlengang als geometrischer Verlauf von Lichtstrahlen ist insbesondere in und/oder um die optische Achse angeordnet und verläuft entlang, konvergierend und/oder dispergierend zu der optischen Achse.

Eine "Kamera" (auch "Kamerakopf" genannt) ist insbesondere ein Gerät zum Empfangen von Bildlicht entlang einer optischen Achse von einem Endoskop und zum Fokussieren des empfangenen Bildlichtes auf mindestens einem Bildsensor. Neben dem mindestens einen Bildsensor kann die Kamera insbesondere eine Blende oder ein Fenster zum Durchlassen des empfangenen Bildlichtes und ein Linsensystem zum Fokussieren des Bildlichtes auf den mindestens einen Bildsensor aufweisen. Die durch mindestens einen Bildsensor aufgenommenen Bilddaten sind insbesondere elektronisch vom Kamerakopf weiter an ein Anzeigesystem und/oder an eine Bildverarbeitungseinheit übertragbar, um das endoskopische Bild für den Benutzer darzustellen. Die Kamera kann Mittel zum Erkennen des verbundenen Endoskops und zum Verarbeiten von Algorithmen aufweisen. Ein Verbinder zum Verbinden eines Endoskops mit der Kamera kann am distalen Ende des Endoskops und/oder dem proximalen Ende des Kamerakopfs angeordnet sein. Auch kann die erfindungsgemäße Filterwechselvorrichtung selbst als Verbinder zum Verbinden eines Endoskops mit einer Kamera ausgebildet sein. Um Vignettierung und somit eine Abschattung am Bildrand zu vermeiden, ist die Filterwechselvorrichtung vorzugsweise im Kameramodul integriert.

Ein "Endoskop" ist insbesondere ein medizinisches oder industrielles Gerät zur endoskopischen Untersuchung und Betrachtung einer menschlichen oder tierischen Körperhöhle und/oder eines industriellen Hohlraums, wie einem Rohr. Das Endoskop weist insbesondere ein Handstück, einen Schaft, eine Lichtquelle, einen Lichtleiter, einen Sensor und/oder eine Kamera auf. Bei dem Endoskop handelt es sich insbesondere um ein Videoendoskop, welches eine digitale Bildaufnahme und Bildübertragung und somit eine integrierte oder verbindbare Kamera aufweist. Neben human- und tiermedizinischen Anwendungen kann ein Endoskop und/oder Videoendoskop auch zu industriellen Zwecken, beispielsweise zur Sichtprüfung in schwer zugänglichen Hohlräumen, eingesetzt werden. Bei industriellen Anwendungen wird ein Endoskop häufig als Boroskop bezeichnet.

Ein "Bildsensor" ist insbesondere ein lichtempfindliches elektronisches Bauelement, welches auf einem inneren Photoeffekt beruht. Mittels des Bildsensors wird insbesondere ein Bild oder werden mehrere Bilder aus dem Betrachtungsbereich der Bildgebungsvorrichtung aufgezeichnet und in elektronische Signale umgewandelt. Der Bildsensor weist eine Sensorebene in der Bildebene des optischen Systems, eines Linsensystems und/oder des Objektivs auf. Bei einem elektronischen Bildsensor kann es sich insbesondere um einen CCD-Sensor (charge-coupled device) oder um einen CMOS-Sensor (complementary metal oxidesemiconductor) handeln.

In einer weiteren Ausführungsform weist die Filterwechselvorrichtung ein zweites Verbindungsrad, ein drittes Verbindungsrad und/oder optional weitere Verbindungsräder und ein drittes Filterrad, ein viertes Filterrad und/oder optional weitere Filterräder auf, wobei das dritte Filterrad, das vierte Filterrad und/oder optional jeweils die weiteren Filterräder mindestens eine erste Eingriffsebene mit vollständig über den jeweiligen Außenumfang gleichmäßig verteilten Zähnen aufweist oder aufweisen.

Somit kann die Anzahl der aufeinanderfolgenden Filterräder entlang der ersten Rotationsachse beliebig skaliert und die Filterwechselvorrichtung erweitert werden. Zwischen dem angetriebenen ersten und/oder endständigen Filterrad und einem gegenüberliegenden zweiten endständigen Rad können somit eine beliebige Anzahl von Mittelrädern angeordnet sein. Diese Mittelräder weisen insbesondere mindestens eine erste Eingriffsebene mit vollständig über den jeweiligen Außenumfang gleichmäßig verteilten Zähnen auf, in welche die Zähne desjenigen Verbindungsrades eingreifen, welches auch in die Zähne des vorherigen Filterrades eingreift. Um bei einer Anwendung der Filterwechselvorrichtung von einer bestehenden Filterposition zu einer neuen Filterposition zu gelangen, sind maximal (2ⁿ)/2-Umdrehungen des ersten angetriebenen und/oder des endständigen Filterrades notwendig. Hierbei kann das erste angetriebene und/oder endständige Filterrad insbesondere im Uhrzeigersinn oder gegen den Uhrzeigersinn gedreht werden. Es ist besonders vorteilhaft, dass trotz einer beliebig großen Anzahl von Filterrädern aufgrund der Aneinanderreihung der Filterräder mit ihren Materialdicken entlang der ersten Rotationsachse ein geringer Bauraum entlang der Rotationsachse und/oder der optischen Achse benötigt ist.

Bei einem dritten, vierten und optional einem weiteren Filterrad handelt es sich prinzipiell um ein oben definiertes Filterrad. Diese dritten, vierten und optional weiteren Filterräder sind jedoch insbesondere als Mittelräder mit einer anderen Anordnung der gleichmäßig entlang des Außenumfangs verteilten Zähne angeordnet. Diese dritten, vierten und optional weiteren Filterräder als Mittelräder weisen insbesondere eine erste Eingriffsebene mit vollständig über den jeweiligen Außenumfang gleichmäßig verteilten Zähnen auf. Bevorzugt weisen alle Zähne der Filterräder und/oder der Verbindungsräder die gleiche Zahngeometrie auf, um eine optimale Übertragung der Bewegung auf das nächstfolgende Verbindungsrad und/oder Filterrad zu gewährleisten. Prinzipiell ist jedoch anzumerken, dass die jeweiligen Zähne der Filterräder und/oder der Verbindungsräder auch eine unterschiedliche Geometrie, beispielsweise Zahnflanken oder Eingriffspunkte, aufweisen können, solange ein Verbindungsrad in die jeweiligen zugehörigen Zähne der Filterräder eingreifen kann.

Das endständige Filterrad, welches gegenüber dem ersten und/oder endständig angetriebenen Filterrad angeordnet ist und mit diesem die dazwischen angeordneten Mittelräder umschließt, kann statt der mindestens einen ersten Eingriffsebene mit vollständig über den jeweiligen Außenumfang gleichmäßig verteilten Zähnen auch Zähne aufweisen, welche über die gesamte Materialdicke dieses endständigen Filterrades ausgebildet sind.

Bei einem zweiten, dritten und optional weiteren Verbindungsrad handelt es sich prinzipiell um ein oben definiertes Verbindungsrad. Bevorzugt sind alle Verbindungsräder geometrisch gleichartig ausgebildet. Jedoch können die Verbindungsräder in ihrer geometrischen Form auch leicht voneinander abweichen, solange das jeweilige Verbindungsrad seine Funktion der Übertragung der Drehbewegung des vorangehenden Filterrades auf das nachfolgende Filterrad bewerkstelligt.

Um den binären mechanischen Übertragungsmechanismus auf das jeweilige nachfolgende Filterrad fortzusetzen, kann oder können das zweite Filterrad, das dritte Filterrad, das vierte Filterrad und/oder optional jeweils die weiteren Filterräder eine zweite Eingriffsebene mit einem Teilabschnitt mit verteilten Zähnen entlang des jeweiligen Außenumfangs aufweisen.

In einer weiteren Ausführungsform der Filterwechselvorrichtung weist oder weisen der Teilabschnitt des ersten Filterrades und/oder der Teilabschnitt der jeweiligen zweiten Eingriffsebene des zweiten Filterrades, des dritten Filterrades, des vierten Filterrades und/oder optional jeweils des weiteren Filterrades Zähne in einem Bereich von 45 % bis 55 %, insbesondere von 48 % bis 52 %, bevorzugt von 50 %, des jeweiligen Außenumfanges auf.

Somit wird bei einer vollen Umdrehung des vorhergehenden Filterrades annähernd oder genau eine halbe Umdrehung auf das nachfolgende Filterrad mittels des zugehörigen Verbindungsrades übertragen, da der zahnlose Abschnitt des ersten Filterrades und/oder der jeweiligen Eingriffsebene eines Filterrades ohne Eingriff und berührungsfrei an dem zugehörigen Verbindungsrad vorbeidreht.

Um redundante Filterkombinationen bereitzustellen und somit ein schnelles Umschalten zwischen verschiedenen Kombinationen zu ermöglichen, kann das jeweilige Filterrad vier Aufnahmen, sechs Aufnahmen und/oder optional weitere geradzahlige Aufnahmen für jeweils einen optischen Filter aufweisen.

Durch eine erhöhte Anzahl von Aufnahmen und somit Plätzen für optische Filter der jeweiligen Filterräder werden bestimmte Filterkombinationen beim Filterwechseln und Weiterdrehen mehrfach bereitgestellt, wodurch ein schnelleres Umschalten und ein Wechseln zwischen verschiedenen Filterkombinationen ermöglicht ist. Prinzipiell kann ausgehend von zwei Filteraufnahmen pro Filterrad die Anzahl an Filteraufnahmen des Filterrades beliebig variiert werden. Jedoch ist eine geradzahlige Anzahl von Aufnahmen erforderlich, da ansonsten aufgrund der Mechanik ein Filter eines nachfolgenden Filterrades durch das vorangehende Filterrad verdeckt werden könnte. Mehr als zwei Filteraufnahmen auf dem jeweiligen Filterrad sind dadurch realisiert, dass sich das jeweilige nachfolgende Filterrad nur ab einer halben Drehung um 180° des vorangehenden Rades bewegt.

In einer weiteren Ausführungsform der Filterwechselvorrichtung weist die Antriebseinheit einen einzigen Motor zum Antreiben des ersten Filterrades oder eines endständigen Filterrades der Filterwechselvorrichtung auf.

Aufgrund der mechanischen, binären Übersetzung und Übertragung der Bewegung des ersten Filterrades oder eines endständigen Filterrades ausgebildet wie oben beschrieben für das erste Filterrad ist als Antrieb nur ein einziger Motor notwendig, um alle Filterräder mittels der Verbindungsräder zu bewegen. Dadurch wird die Standzeit der Filterwechselvorrichtung erhöht und der Verschleiß und der Bauraum verringert, da nicht jedes Filterrad mit einem eigenen Motor angetrieben werden muss. Aufgrund der mechanischen, binären Übertragung zwischen den Filterrädern können mit nur einem Motor gewünschte Filterkombinationen parallel im Strahlengang erreicht und sogar redundant bereitgestellt werden. Hierbei kann der Motor eine Welle verbunden mit dem ersten und/oder endständigen Filterrad antreiben.

Um einen Antrieb über den Außenumfang des ersten und/oder endständigen Filterrades zu realisieren, kann das erste Filterrad oder das endständige Filterrad einen Antriebsabschnitt mit vollständig über den jeweiligen Außenumfang verteilten Zähnen aufweisen, sodass das erste Filterrad oder das endständige Filterrad über ein Antriebszahnrad mittels des einen einzigen Motors antreibbar ist.

In einer weiteren Ausführungsform der Filterwechselvorrichtung weist das erste Filterrad oder das endständige Filterrad eine Welle oder eine Aufnahme für eine Welle entlang der ersten Rotationsachse zum Verbinden mit dem einen einzigen Motor auf.

Um die Anzahl der Bauteile der Filterwechselvorrichtung gering zu halten, können die Verbindungsräder eine gemeinsame zweite Rotationsachse aufweisen.

In einer weiteren Ausführungsform der Filterwechselvorrichtung ist oder sind die erste Rotationsachse und/oder die zweite Rotationsachse an der Grundplatte angeordnet.

Um einen exakten Haltemechanismus bereitzustellen in dem Zeitraum, in dem das jeweilige Filterrad nicht mit seinen Zähnen in ein Verbindungsrad eingreift, kann die Filterwechselvorrichtung eine Halteeinrichtung zum Halten eines jeweils sich nicht drehenden Filterrades in seiner Position beim Antreiben aufweisen.

Bei einer "Halteeinrichtung" kann es sich insbesondere um eine Einrichtung und/oder ein Bauteil handeln, welches ein Halten eines sich nicht drehenden Filterrades oder mehrerer Filterräder in seiner jeweiligen Ruheposition gewährleistet. Die Halteeinrichtung kann beispielsweise mittels Magnetkraft, einer Feder, Reibung und/oder einem anderen Mechanismus realisiert sein.

Somit kann, während ein zahnloser Abschnitt eines jeweiligen Filterrades an dem zugehörigen Verbindungsrad ohne Eingreifen vorbeidreht, ein Rotieren des nachfolgenden Filterrades verhindert werden, indem dieses mechanisch durch die Halteeinrichtung, beispielsweise ein federndes Druckstück, in und/oder an seiner Position gehalten wird. Zudem kann durch ein Abstandsstück oder eine andere konstruktive Ausgestaltung eine direkte, reibschlüssige Berührung der Radflächen der aufeinanderfolgenden Filterräder verhindert sein.

In einem weiteren Aspekt der Erfindung wird die Aufgabe gelöst durch einen Kamerakopf für ein Endoskop, wobei der Kamerakopf einen Bildsensor, eine Öffnung zum Empfangen von Licht eines Bildes entlang eines optischen Pfades und ein optisches Linsensystem zur Fokussierung des Lichtes auf den Bildsensor aufweist, wobei der Kamerakopf zumindest eine zuvor beschriebene Filterwechselvorrichtung aufweist.

Somit wird ein Kamerakopf bereitgestellt, an dem oder in dem eine kompakte, platzsparend ausgebildete Filterwechselvorrichtung angeordnet ist. Im Falle der Anordnung zumindest einer Filterwechselvorrichtung direkt in dem Kamerakopf kann der Kamerakopf lösbar mit verschiedenen Arten von Endoskopen verbunden werden. Selbstverständlich kann der Kamerakopf auch zwei oder mehrere Filterwechselvorrichtungen in Reihe in einem optischen Pfad und/oder entlang der optischen Achse aufweisen, auch wenn eine einzige Filterwechselvorrichtung aufgrund der parallelen Anordnung von beliebig vielen Filterrädern und damit Filtern bereits eine vielfältige Auswahl an Filterkombinationen, verschiedene mögliche Filtereinstellungen und -anwendungen realisiert. Somit werden mittels des Kamerakopfes unterschiedliche multispektrale Bildgebungen und/oder eine breite Anwendung von verschiedenen Fluorophoren in der Fluoreszenzbildgebung ermöglicht.

Um eine derzeitig verwendete Filterkonfiguration oder diese für gewünschte unterschiedliche Beobachtungsmodi anzupassen, kann der Kamerakopf eine Detektionseinheit zum Detektieren einer Identifikation des jeweiligen optischen Filters in dem optischen Pfad aufweisen. Ebenso kann der Kamerakopf eine Kontrolleinheit zum Anpassen der Drehgeschwindigkeit der Filterplatte und zum Überprüfen und/oder Anpassen des jeweiligen optischen Filters angeordnet im Strahlengang entsprechend des jeweils gewählten Betriebsmodus aufweisen.

In einem weiteren Aspekt der Erfindung wird die Aufgabe gelöst durch einen Nachrüstsatz zum Nachrüsten eines Kamerakopfes und/oder eines Endoskops, wobei der Nachrüstsatz zumindest eine zuvor beschriebene Filterwechselvorrichtung aufweist, sodass die Filterwechselvorrichtung zwischen einem proximalen Ende des Endoskops und einem distalen Ende des Kamerakopfs anordenbar ist.

Somit wird ein Nachrüstsatz (auch als "Adapter" bezeichnet) mit mindestens einer Filterwechselvorrichtung bereitgestellt, welcher gleichzeitig als Verbinder zwischen einem bereits existierenden Endoskop und einem existierenden Kamerakopf als auch zum Ermöglichen von unterschiedlichen Beobachtungsmodi dient. Zusätzlich kann der Nachrüstsatz auch zwei oder mehrere Filterwechselvorrichtungen aufweisen, welche in Reihe zwischen dem Endoskop und dem Kamerakopf angeordnet sind oder eine Filterwechselvorrichtung kann durch eine andere erfindungsgemäße Filterwechselvorrichtung zum Ermöglichen von verschiedenen Anwendungen und/oder Beobachtungsoptionen ersetzt werden.

Im Weiteren wird die Erfindung anhand von Ausführungsbeispielen näher erläutert. Es zeigen
- Figur 1: eine schematische dreidimensionale Ausschnittsansicht eines Endoskopsystems mit einem Endoskop, einem Filterwechsler und einem Kamerakopf,
- Figur 2: eine dreidimensionale Darstellung eines Filterwechslers in Seitenansicht mit einer Endoskopaufnahme und einer Kameraaufnahme,
- Figur 3: eine dreidimensionale Darstellung des geöffneten Filterwechslers aus Figur 2 mit einer Innenansicht,
- Figur 4: eine dreidimensionale Darstellung eines ersten Filterzahnrades,
- Figur 5: eine dreidimensionale Darstellung eines zweiten Filterzahnrades,
- Figur 6: eine dreidimensionale Darstellung eines dritten Filterzahnrades,
- Figur 7: eine dreidimensionale Darstellung eines Verbindungszahnrades, und
- Figur 8: eine dreidimensionale Darstellung des Filterwechslers bei geöffnetem Gehäuse mit den Filterzahnrädern und den Verbindungszahnrädern.

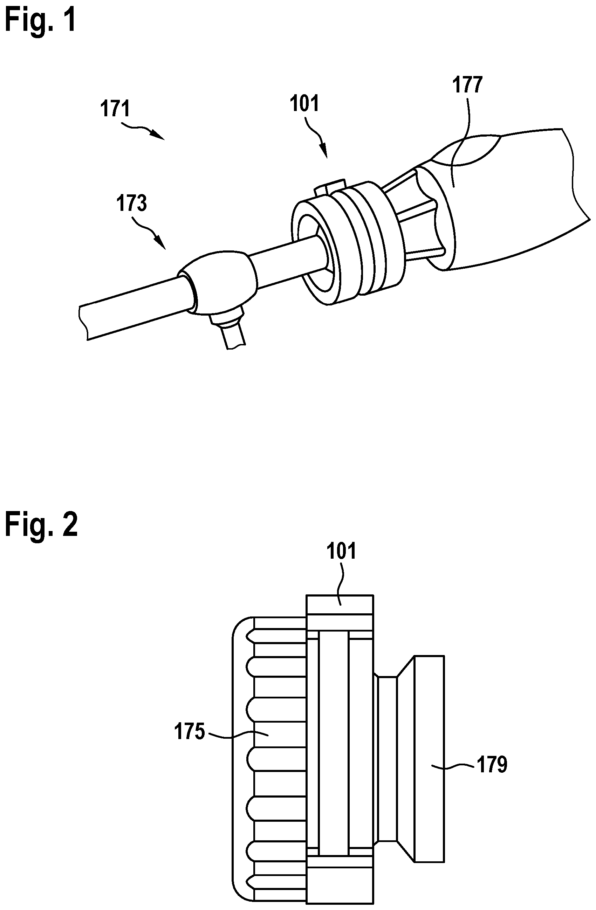

Ein Endoskopsystem 171 weist einen Kamerakopf 177, einen Filterwechsler 101 und ein Endoskop 173 auf. Der Filterwechsler 101 ist mittels einer Kameraaufnahme 179 an dem Kamerakopf 177 und mittels einer Endoskopaufnahme 175 an dem Endoskop 173 verbunden (Figuren 1 und 2). Des Weiteren weist der Filterwechsler 101 ein Gehäuse 103 mit einer als distaler Deckel ausgebildeten Grundplatte 107 auf. Die Grundplatte 107 ist außenliegend mittels Schrauben 105 mit einem proximalen Deckel des Gehäuses 103 verbunden.

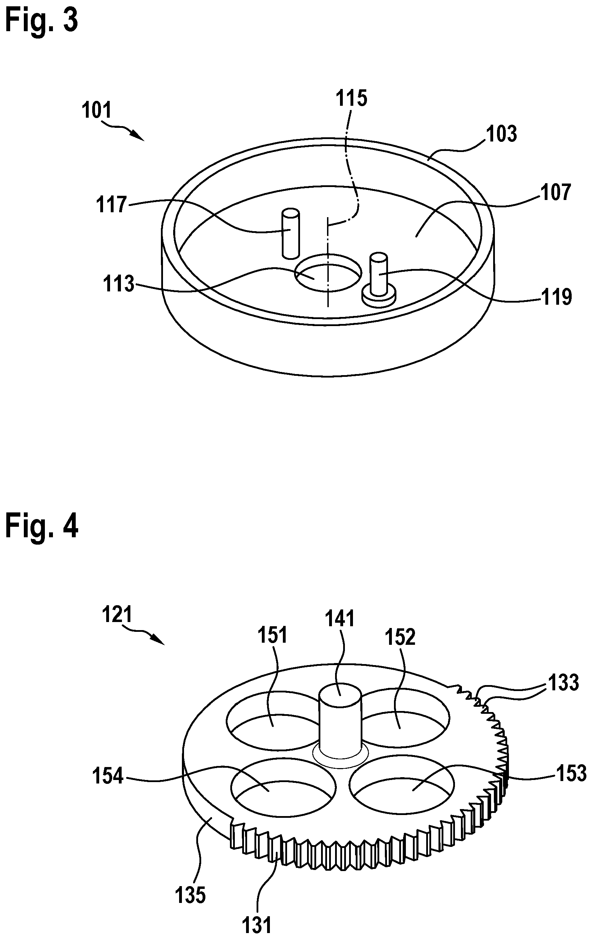

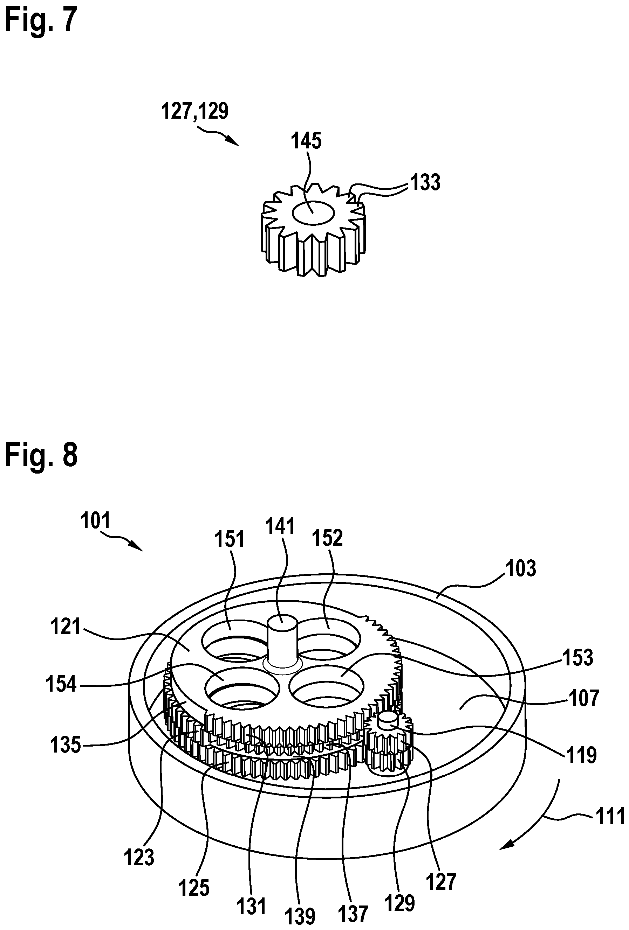

Innen im Gehäuse 103 weist der Filterwechsler 101 ein erstes Filterzahnrad 121, ein zweites Filterzahnrad 123 und ein drittes Filterzahnrad 125 auf, wobei das dritte Filterzahnrad 125 oberhalb der Grundplatte 107 angeordnet ist. Des Weiteren weist der Filterwechsler 101 ein erstes Verbindungszahnrad 127 und ein zweites Verbindungszahnrad 129 auf (Figur 8). Durch die Grundplatte 107 ist durchgehend ein optischer Durchgang 113 mit einer optischen Achse 115 ausgebildet. Der optische Durchgang 113 und die optische Achse 115 sind mittig und zentriert zu einem Außendurchmesser der Grundplatte 107 und des Gehäuses 103 angeordnet. Innen im Gehäuse 103 weist die Grundplatte 107 eine verbundene erste Rotationsachse 117 und eine zweite Rotationsachse 119 auf (Fig. 3).

Das erste Filterzahnrad 121 weist eine erste Filteraufnahme 151, eine zweite Filteraufnahme 152, eine dritte Filteraufnahme 153 und eine vierte Filteraufnahme 154 auf, welche beabstandet zu einer mittigen Welle 141 und einem Außendurchmesser des ersten Filterzahnrades angeordnet sind. In diese vier Filteraufnahmen 151, 152, 153 und 154 sind jeweils nicht gezeigte unterschiedliche optische Filter eingesetzt. An seiner Außenseite weist das erste Filterzahnrad 121 einen Teilabschnitt 131 mit Zähnen 133 auf, welcher 50 % des Außenumfanges des ersten Filterzahnrades 121 einnimmt und somit einen Kreisbogen von 180° umschreibt. Die anderen 50 % des Außenumfanges des ersten Filterrades 121 sind als eine Aussparung 135 frei von Zähnen ausgebildet. Die Welle 141 weist innenliegend an einer Unterseite und somit gegenüber der in Figur 4 gezeigten oberen Radfläche eine Bohrung zur Aufnahme der ersten Rotationsachse 117 auf.

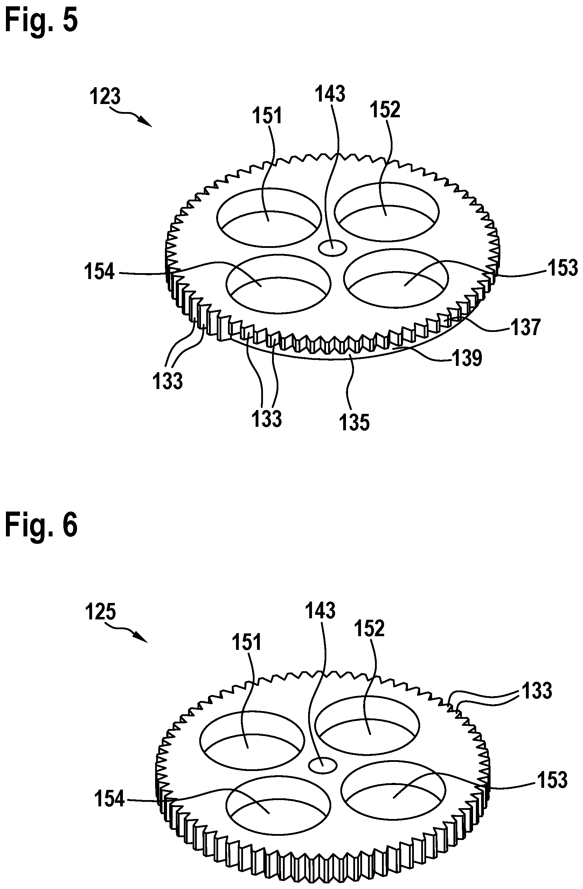

Das zweiter Filterzahnrad 123 weist ebenfalls vier Filteraufnahmen 151, 152, 153 und 154 auf. In der Mitte des zweiten Filterzahnrades 123 ist eine durchgehende Bohrung 143 für die erste Rotationsachse 117 ausgebildet. An seinem Außenumfang weist das zweite Filterzahnrad 123 außen angrenzend an der in Figur 5 gezeigten oberen Radfläche eine erste Eingriffsebene 137 mit rundumlaufenden Zähnen 133 auf. Unterhalb dieser ersten Eingriffsebene 137 weist eine zweite Eingriffsebene 139 einen Teilabschnitt mit einer Aussparung 135 entlang von 50 % des Außenumfanges des zweiten Filterzahnrades 123 auf, wobei eine andere Hälfte des Außenumfanges mit durchgehenden Zähnen 133 über die erste Eingriffsebene 137 und die zweite Eingriffsebene 139 ausgebildet ist.

Das dritte Filterzahnrad 125 weist ebenfalls vier Filteraufnahmen 151, 152, 153 und 154 sowie eine mittige Bohrung 143 zur Aufnahme der ersten Rotationsachse 117 auf. Das dritte Filterzahnrad 125 weist rundumlaufend und entlang seiner Materialdicke durchgehende Zähne 133 auf.

Bei einer vorhergehenden Montage des Filterwechslers 101 ist das dritte Filterzahnrad 125 mit seiner Bohrung 143 auf die erste Rotationsachse 117 gesteckt worden, sodass die Unterseite des dritten Filterzahnrads 125 an der innenliegenden Seite der Grundplatte 107 liegt. Anschließend ist das zweite Filterzahnrad 125 wie in Figur 5 gezeigt mit seiner Oberseite nach oben ausgerichtet ebenfalls mit seiner Bohrung 143 auf die erste Rotationsachse 117 gesteckt worden, sodass die Unterseite des zweiten Filterzahnrades 123 auf der Oberseite des dritten Filterzahnrades 125 aufliegt. Abschließend ist das erste Filterzahnrad 121 mit seiner innenliegend in der Welle 141 eingebrachten Bohrung auf die erste Rotationsachse 117 gesteckt worden, sodass die Unterseite des ersten Filterzahnrades 121 auf der Oberseite des zweiten Filterzahnrades 123 aufliegt. Die erste Rotationsachse 117 ist derart beabstandet zur optischen Achse 115 und zum optischen Durchgang 113 angeordnet, dass jede der vier Filteraufnahmen 151, 152, 153 und 154 konzentrisch in den optischen Durchgang 113 und zur optischen Achse 115 einschwenkbar und anordenbar ist.

Das erste Verbindungszahnrad 127 und das zweite Verbindungszahnrad 129 sind gleichartig mit einer mittigen Bohrung 145 für die zweite Rotationsachse 119 und mit über ihre Außendurchmesser und ihre Höhe durchgängig ausgebildeten Zähnen ausgebildet. Das erste Verbindungszahnrad 127 ist auf dem zweiten Verbindungszahnrad 129 um die zweite Rotationsachse 119 angeordnet (Figuren 7 und 8). Hierbei stehen die Zähne 133 des ersten Verbindungszahnrades 127 mit den Zähnen 133 des ersten Filterzahnrades 121 und des zweiten Filterzahnrades 123 in Eingriff, während die Zähne 133 des zweiten Verbindungszahnrades 129 in die Zähne 133 des dritten Filterzahnrades 125 eingreifen. Somit kombiniert das erste Verbindungszahnrad 127 den Teilabschnitt 131 mit Zähnen 133 des ersten Filterzahnrads 121 für eine Bewegungsübertragung mit der ersten Eingriffsebene 137 mit vollständig umlaufenden Zähnen 133 und das zweite Verbindungsrad 129 verbindet die zweite Eingriffsebene 139 des zweiten Filterzahnrades 123 mit dem Abschnitt mit 50 % der umlaufenden Zähne 133 mit den Zähnen 133 des dritten Filterzahnrades 125.

Mit dem Filterwechsler 101 und dem Endoskopsystem 171 werden folgende Arbeitsgänge durchgeführt.

Die Welle 141 wird proximalseitig mittels eines nicht in den Figuren gezeigten einzigen Motors angetrieben und dadurch das erste Filterzahnrad 121 in der Rotationsrichtung 111 im Uhrzeigersinn gedreht. Solange die Zähne 133 des ersten Verbindungszahnrades 127 in die Zähne 133 des Teilabschnitts 131 des ersten Filterzahnrades 121 eingreifen, bewegt sich das erste Verbindungszahnrad 127 um die zweite Rotationsachse 119. Greift das erste Verbindungszahnrad 127 gleichzeitig in die Zähne 133 der ersten Eingriffsebene 137 mit den vollständig rundumlaufenden Zähnen 133 des zweiten Filterzahnrades 123 ein, so wird entsprechend auch das zweite Filterzahnrad 123 in Rotationsrichtung 111 gedreht. Da alle drei Filterzahnräder 121, 123 und 125 nur auf der ersten Rotationsachse 117 gelagert sind, erfolgt die Bewegungsübertragung von dem ersten angetriebenen Filterzahnrad 121 auf das zweite Filterzahnrad 123 und das nachfolgende dritte Filterzahnrad 125 nur über die beiden Verbindungszahnräder 127 und 129. Dementsprechend wird bei einer vollständigen Umdrehung des ersten Filterzahnrades 121 in Rotationsrichtung 111 aufgrund des ersten Teilabschnittes 131 mit Zähnen 133 das zweite Filterzahnrad 123 mittels des ersten Verbindungsrads 127 um eine halbe Umdrehung gedreht, da beim Vorliegen des ersten Verbindungszahnrades 127 im Bereich der Aussparung 135 des ersten Filterzahnrades 121 kein Eingreifen der Zähne 133 des ersten Verbindungszahnrades 127 erfolgt. Somit wird das erste Verbindungszahnrad 127 während einer halben Umdrehung des ersten Filterzahnrades 121 nicht gedreht. Bei einer vollen Umdrehung des zweiten Filterzahnrades 123 wird mittels des zweiten Verbindungszahnrads 129 ebenso das dritte Filterzahnrad 125 um eine halbe Umdrehung gedreht, solange die Zähne 133 des zweiten Verbindungszahnrades 129 in die 50 % umlaufenden Zähne 133 der zweiten Eingriffsebene 139 des zweiten Filterzahnrades 123 eingreifen.

Durch jeweils eine Vierteldrehung des angetriebenen ersten Filterzahnrades 121 werden nachfolgend die erste Filteraufnahme 151, die zweite Filteraufnahme 152, die dritte Filteraufnahme 153 und die vierte Filteraufnahme 154 in den optischen Durchgang 113 eingebracht. In die vier Filteraufnahmen 151 bis 154 des ersten Filterzahnrades 121 ist jeweils ein Filter A, B, C und D eingebracht. Ebenso sind diese vier Filter A, B, C und D in die entsprechenden Filteraufnahmen 151 bis 154 des zweiten Filterzahnrades 123 und des dritten Filterzahnrades 125 eingesetzt. Dadurch ergeben sich die in der Tabelle 1 gezeigten Filterkombinationen. Bei einem oben beschriebenen Filterwechsler 101 mit den drei Filterzahnrädern 121, 123 und 125 und den vier Filteraufnahmen 151 bis 154 sind alle in der Tabelle 1 gezeigten Filterkombinationen möglich. Weisen die drei Filterzahnräder 121, 123 und 125 jeweils nur zwei Filteraufnahmen auf, so sind nur die mit einem vorangestellten Stern markierten Filterpositionskombinationen realisierbar.

**Tabelle 1: Kombinationsmöglichkeiten bei jeweils vier Filter A, B, C, D der Filterzahnräder 121, 123, 125**

| | **Filterzahnrad 125** | **Filterzahnrad 123** | **Filterzahnrad 121** | **Drehwinkel Filterzahnrad 121** |
|---|---|---|---|---|
| * | A | A | A | 0° |
| | A | A | B | 90° |
| * | A | A | C | 180° |
| | A | B | D | 270° |
| * | A | C | A | 360° |
| | A | C | B | 450° |
| * | A | C | C | 540° |
| | B | D | D | 630° |
| * | C | A | A | 720° |
| | C | A | B | 810° |
| * | C | A | C | 900° |
| | C | B | D | 990° |
| * | C | C | A | 1080° |
| | C | C | B | 1170° |
| * | C | C | C | 1260° |
| | D | D | D | 1350° |

In einer Alternative des Filterwechslers 101 weist der Filterwechsler 101 nur das erste Filterzahnrad 121 und das dritte Filterzahnrad 125 als endständige Filterzahnräder sowie ein verbindendes erstes Verbindungszahnrad 127 auf. Die vier Filteraufnahmen 151 bis 154 des ersten Filterzahnrads 121 und des dritten Filterzahnrads 125 sind mit einer beispielhaften Filterauswahl bestückt (Tabelle 2). Diese Alternative des Filterwechslers 101 mit nur dem ersten Filterzahnrad 121 und dem dritten Filterzahnrad 125 wird prinzipiell wie oben beschrieben betrieben. Dadurch sind mittels angetriebener Drehung des ersten Filterzahnrads 121 die in Tabelle 3 gezeigten Filterkombinationen einstellbar. Somit sind auch mit der Alternative des Filterwechslers 101 mit nur den beiden Filterzahnrädern 121 und 125 alle möglichen Filterkombinationen erreichbar und einstellbar. Wie ein Vergleich der Filterkombinationen der Tabelle 3 mit der Tabelle 1 zeigt, hat der oben beschriebene Filterwechsler mit den drei Filterzahnrädern 121, 123 und 125 jedoch den Vorteil, dass wiederholende, redundante Filterkombinationen bereitgestellt werden, wodurch eine Umdrehungslänge und somit die Schaltdistanz und die Wechselzeit zwischen besonders wichtigen und häufig verwendeten Filterkombinationen verringert wird.

**Tabelle 2: Beispiel einer möglichen Filterauswahl eines Filterwechslers mit zwei Filterzahnrädern**

| **Filterzahnrad** | **Filter A** | **Filter B** | **Filter C** | **Filter D** |
|---|---|---|---|---|
| 125 | Kein Filter | Rotfilter | Weißfilter | NIR-Filter |
| 121 | Weißfilter | Blaufilter | Kein Filter | Kein Filter |

**Tabelle 3: Kombinationsmöglichkeiten bei jeweils vier Filter A, B, C, D bei zwei Filterzahnräder**

| **Filterzahnrad 125** | **Filterzahnrad 121** | **Kombination** |
|---|---|---|
| A | A | Kein Filter, Weißfilter |
| A | B | Kein Filter, Blaufilter |

| | | |
|---|---|---|
| A | C | Kein Filter, Kein Filter |
| B | D | Rotfilter, Kein Filter |
| C | A | Weißfilter, Weißfilter |
| C | B | Weißfilter, Blaufilter |
| C | C | Weißfilter, Kein Filter |
| D | D | NIR-Filter, Kein Filter |

Somit wird ein Filterwechsler 101 bereitgestellt, mit dem schnell, effizient und präzise mindestens zwei verschiedene Filter mittels der Filterzahnräder 121, 123, 125 in den optischen Strahlengang parallel einschwenkbar sind und welcher mit verschiedenen Arten von Endoskopen 173 und Kameraköpfen 177 einsetzbar ist.

### Bezugszeichenliste

- 101: Filterwechsler
- 103: Gehäuse
- 107: Grundplatte
- 111: Rotationsrichtung
- 113: optischer Durchgang
- 115: optische Achse
- 117: erste Rotationsachse
- 119: zweite Rotationsachse
- 121: erstes Filterzahnrad
- 123: zweites Filterzahnrad
- 125: drittes Filterzahnrad
- 127: erstes Verbindungszahnrad
- 129: zweites Verbindungszahnrad
- 131: Teilabschnitt mit Zähnen
- 133: Zahn
- 135: Aussparung
- 137: erste Eingriffsebene
- 139: zweite Eingriffsebene
- 141: Welle
- 143: Bohrung für erste Rotationsachse
- 145: Bohrung für die zweite Rotationsachse
- 151: erste Filteraufnahme
- 152: zweite Filteraufnahme
- 153: dritte Filteraufnahme
- 154: vierte Filteraufnahme
- 171: Endoskopsystem
- 173: Endoskop
- 175: Endoskopaufnahme
- 177: Kamerakopf
- 179: Kameraaufnahme

## Patentansprüche

1. Filterwechselvorrichtung (101) für eine endoskopische Kamera, wobei die Filterwechselvorrichtung (101) eine Grundplatte (107), einen optischen Durchgang (113) mit einer optischen Achse (115), mindestens ein erstes drehbares Filterrad (121), ein zweites drehbares Filterrad (123) und ein drehbares erstes Verbindungsrad (127) sowie eine Antriebseinheit zum Antreiben eines der Filterräder (121, 123) aufweist, wobei das erste Filterrad (121) und das zweite Filterrad (123) jeweils mindestens zwei Aufnahmen (151, 152, 153, 154) für jeweils einen optischen Filter aufweisen, die Filterräder (121, 123) eine gemeinsame erste Rotationsachse (117) aufweisen, das zweite Filterrad (121) eine erste Eingriffsebene (137) mit vollständig über seinem Außenumfang verteilten Zähnen (133) aufweist und das erste Verbindungsrad (127) als Zahnrad mit vollständig über seinem Außenumfang verteilten Zähnen (133) ausgebildet ist, **dadurch gekennzeichnet, dass** das erste Filterrad (121) einen Teilabschnitt (131) mit verteilten Zähnen (133) entlang seines Außenumfanges aufweist und das erste Verbindungsrad (127) an dem Außenumfang des ersten Filterrades (121) und an dem Außenumfang des zweiten Filterrades (123) derart angeordnet ist, dass die Zähne (133) des Teilabschnittes (131) des ersten Filterrades (121) in die Zähne (133) der ersten Verbindungsrades (127) zum Drehen des ersten Verbindungsrades (127) und die Zähne (133) des ersten Verbindungsrades (127) in die Zähne (133) der ersten Eingriffsebene (137) des zweiten Filterrades (123) eingreifbar sind, sodass bei dem Antreiben des ersten Filterrades (121) mittels der Antriebseinheit mindestens jeweils eine Aufnahme (151, 152, 153, 154) des ersten Filterrades (121) und des zweiten Filterrades (123) parallel in den optischen Durchgang (113) positionierbar sind.

2. Filterwechselvorrichtung (101) nach Anspruch 1, **dadurch gekennzeichnet, dass** die Filterwechselvorrichtung (101) ein zweites Verbindungsrad (129), ein drittes Verbindungsrad und/oder optional weitere Verbindungsräder und ein drittes Filterrad (153), ein viertes Filterrad und/oder optional weitere Filterräder aufweist, wobei das dritte Filterrad (153), das vierte Filterrad und/oder optional jeweils die weiteren Filterräder mindestens eine erste Eingriffsebene (137) mit vollständig über dem jeweiligen Außenumfang gleichmäßig verteilten Zähnen (133) aufweist oder aufweisen.

3. Filterwechselvorrichtung (101) nach Anspruch 1 oder 2, **dadurch gekennzeichnet, dass** das zweite Filterrad (123), das dritte Filterrad (125), das vierte Filterrad und/oder optional jeweils die weiteren Filterräder eine zweite Eingriffsebene (139) mit einem Teilabschnitt (131) mit verteilten Zähnen (133) entlang des jeweiligen Außenumfanges aufweist oder aufweisen.

4. Filterwechselvorrichtung (101) nach einem der vorherigen Ansprüche, **dadurch gekennzeichnet, dass** der Teilabschnitt (131) des ersten Filterrades (121) und/oder der Teilabschnitt (131) der jeweiligen zweiten Eingriffsebene (139) des zweiten Filterrades (123), des dritten Filterrades (125), des vierten Filterrades und/oder optional jeweils des weiteren Filterrades Zähne (133) in einem Bereich von 45 % bis 55 %, insbesondere von 48 % bis 52 %, bevorzugt von 50 %, des jeweiligen Außenumfanges aufweist oder aufweisen.

5. Filterwechselvorrichtung (101) nach einem der vorherigen Ansprüche, **dadurch gekennzeichnet, dass** das jeweilige Filterrad (121, 123, 125) vier Aufnahmen (151, 152, 153, 154), sechs Aufnahmen und/oder optional weitere geradzahlige Aufnahmen für jeweils einen optischen Filter aufweist.

6. Filterwechselvorrichtung (101) nach einem der vorherigen Ansprüche, **dadurch gekennzeichnet, dass** die Antriebseinheit einen einzigen Motor zum Antreiben des ersten Filterrades (121) oder eines endständigen Filterrades der Filterwechselvorrichtung aufweist.

7. Filterwechselvorrichtung (101) nach Anspruch 6, **dadurch gekennzeichnet, dass** das erste Filterrades (121) oder das endständige Filterrad einen Antriebsabschnitt mit vollständig über dem jeweiligen Außenumfang verteilten Zähnen aufweist, sodass das erste Filterrad (121) oder das endständige Filterrad über ein Antriebszahnrad mittels des einen einzigen Motors antreibbar ist.

8. Filterwechselvorrichtung (101) nach Anspruch 6, **dadurch gekennzeichnet, dass** das erste Filterrad (121) oder das endständige Filterrad eine Welle (141) oder eine Aufnahme für eine Welle entlang der ersten Rotationsachse (117) zum Verbinden mit dem einen einzigen Motor aufweist.

9. Filterwechselvorrichtung (101) nach einem der Ansprüche 2 bis 8, **dadurch gekennzeichnet, dass** die Verbindungsräder (127, 129) eine gemeinsame zweite Rotationsachse (119) aufweisen.

10. Filterwechselvorrichtung (101) nach einem der vorherigen Ansprüche, **dadurch gekennzeichnet, dass** die erste Rotationsachse (117) und/oder die zweite Rotationsachse (119) an der Grundplatte (107) angeordnet ist oder sind.

11. Filterwechselvorrichtung (101) nach einem der vorherigen Ansprüche, **dadurch gekennzeichnet, dass** die Filterwechselvorrichtung (101) eine Halteeinrichtung zum Halten eines jeweils sich nicht drehenden Filterrades (125) in seiner Position beim Antreiben aufweist.

12. Kamerakopf (177) für eine Endoskop (173), wobei der Kamerakopf (177) einen Bildsensor, eine Öffnung zum Empfangen von Licht eines Bildes entlang eines optischen Pfades und ein optisches Linsensystem zum Fokussieren des Lichtes auf den Bildsensor aufweist, **dadurch gekennzeichnet, dass** der Kamerakopf (177) zumindest eine Filterwechselvorrichtung (101) nach einem der Ansprüche 1 bis 11 aufweist.

13. Nachrüstsatz zum Nachrüsten eines Kamerakopfes und/oder eines Endoskops, **dadurch gekennzeichnet, dass** der Nachrüstsatz zumindest eine Filterwechselvorrichtung (101) nach einem der Ansprüche 1 bis 11 aufweist, sodass die Filterwechselvorrichtung (101) zwischen einem proximalen Ende des Endoskops (173) und einem distalen Ende des Kamerakopfes (177) anordenbar ist.
